# EUROPEAN PATENT APPLICATION

(11) **EP 2 280 029 A1**
(43) Date of publication of application: **02.02.2011**
(21) Application number: 09754703.8
(22) Date of filing: 26.05.2009
(51) Int. Cl.: C07K 14/47

(54) **METHOD FOR EFFICIENTLY AMPLIFYING ABNORMAL PRION PROTEIN DERIVED FROM BSE**

(30) Priority: 28.05.2008 JP 2008139279
(71) Applicant: Incorporated Administrative Agency National Agriculture and Food Research Organization, Ibaraki 305-8517 (JP)
(72) Inventor: MURAYAMA, Yuichi, Tsukuba-shi Ibaraki 305-0856 (JP); YOSHIOKA, Miyako, Tsukuba-shi Ibaraki 305-0856 (JP); MASUJIN, Kentaro, Tsukuba-shi Ibaraki 305-0856 (JP); OKADA, Hiroyuki, Tsukuba-shi Ibaraki 305-0856 (JP); IWAMARU, Yoshifumi, Tsukuba-shi Ibaraki 305-0856 (JP); IMAMURA, Morikazu, Tsukuba-shi Ibaraki 305-0856 (JP); MATSUURA, Yuichi, Tsukuba-shi Ibaraki 305-0856 (JP); YOKOYAMA, Takashi, Tsukuba-shi Ibaraki 305-0856 (JP); MOHRI, Shirou, Tsukuba-shi Ibaraki 305-0856 (JP)
(74) Representative: Hiebl, Inge Elisabeth
(86) International application number: PCT/JP2009/059618
(87) International publication number: WO 2009/145195

(57) **Abstract**

A method for efficiently amplifying abnormal prion protein (PrP^{Sc}) derived from bovine spongiform encephalopathy (BSE) is provided. Ultimately, the invention aims at eradicating the transmission of a prion disease by detecting a BSE-infected cow early and developing a method for inactivating prions and permitting early examination of prion inactivation. Provided is a method for efficiently amplifying PrP^{Sc} derived from BSE, wherein the method is based on a PMCA (protein misfolding cyclic amplification) method in which normal prion protein (PrP^{C}) is used as a source and PrP^{Sc} is used as a seed, and PrP^{Sc} derived from BSE is amplified by stir-mixing, incubating, and sonicating both the PrP^{C} and the PrP^{Sc} repeatedly, and wherein the method includes performing stir-mixing-incubation-sonication in the presence of a polysaccharide sulfate.

## Description

### TECHNICAL FIELD

The present invention relates to in vitro methods for efficiently amplifying abnormal prion protein derived from bovine spongiform encephalopathy (BSE).

### BACKGROUND ART

The first disease discovered as one of the diseases caused by propagation of abnormal prion protein is scrapie, which is a disease associated with ataxia found in sheep and is characterized by spongy vacuolation in the brain. Later, bovine spongiform encephalopathy (BSE) also referred to as mad cow disease, Creutzfeldt Jakob disease (CJD) in humans, Gerstmann-Straussler-Scheinker syndrome (GSS) or the like became known as the diseases caused by propagation of abnormal prion protein.

These diseases are not caused by viral infection and already-known pathogens have not been discovered. A specific protein is commonly found in these diseases, and thus is believed to be an etiologic agent that causes transmission and infection. A "proteinaceous infectious particle: prion" has been proposed and the specific diseases described above have been called prion diseases.

Incidentally, it has been found that the human prion protein gene is on chromosome 20 and a prion protein is composed of 235 amino acids. The main component of the infectious agent, prion, is believed to be this prion protein. The prion protein that the infectious agent is composed of is called a scrapie form or abnormal form of prion protein (PrP^{Sc}), and a normal form of prion protein is called normal prion protein (PrP^{C}).
Since prion diseases have been found in many animal species including humans, and the accumulation of abnormal prion protein (PrP^{Sc})-, which is generally resistant to proteases, is observed in infected individuals, this PrP^{Sc} is believed to be involved in the diseases as a main etiologic agent.

Transmission of prion diseases may occur between different animal species. For example, cows might have been infected with scrapie via animal feed containing contaminated materials derived from sheep infected with scrapie and consequently have developed BSE. Furthermore, it has been thought that cats that ate feed containing materials derived from cows infected with BSE developed feline spongiform encephalopathy (FSE). Accordingly, recent research has reported that BSE, in particular, has a high potential to infect humans (Non-Patent Document 1).

Bovine spongiform encephalopathy (BSE) was first reported in the United Kingdom in 1986, and 35 cases have been identified in Japan since 2001.
At present, such simple diagnostic methods as used for diagnosis of other infectious diseases and metabolic diseases cannot be applied to animals infected with abnormal prion protein (PrP^{Sc}), and therefore, a practical ante-mortem diagnostic method for BSE has not been established. Since only a trace amount of PrP^{Sc} is expected to exist in biological materials such as blood, a supersensitive detection technique with a detection limit far superior to that of conventional methods needs to be developed for an ante-mortem diagnostic method.

Abnormal prion protein (PrP^{Sc}) also has a feature in that it is not inactivated by ordinal sterilization. A bioassay is generally used to determine inactivation of PrP^{Sc}, and the bioassay includes inoculating a laboratory animal such as a mouse with PrP^{Sc} that is thought to have been inactivated and determining whether the mouse develops symptoms or not, thereby detecting the infectivity of PrP^{Sc}. However, this assay requires long-term breeding and observation of a laboratory animal and the result is only obtained after several tens to several hundreds of days, and therefore it is problematic in terms of the enormous amount of time and money required for follow-up.
Accordingly, if a highly sensitive method by which PrP^{Sc} remaining after inactivation can be detected in a short period of time is successfully developed, the development of a method for inactivating prion and the examination of prion inactivation will be substantially improved.

One of the conventional methods developed to detect abnormal prion protein (PrP^{Sc}) is a PMCA (protein misfolding cyclic amplification) method where PrP^{Sc} is amplified by mixing a brain homogenate infected with prion and a normal brain homogenate in vitro and repeating sonication and incubation with stirring. The PMCA method enabled detection of a trace amount of PrP^{Sc} (Non-Patent Document 2 and Patent Document 1).

PrP^{Sc} used in the PMCA method derived from a brain homogenate derived from a hamster infected with scrapie (hamster-adapted 263K strain). The PMCA method is a method for amplifying PrP^{Sc} by repeating a series of mixing-incubation-sonication cycles, wherein the method includes diluting the above mentioned brain homogenate, adding a brain homogenate derived from a normal hamster, which serves as normal prion protein (PrP^{C}), to the diluted brain homogenate and mixing them together, incubating the mixture *in vitro,* allowing conformational conversion of excessively added PrP^{C} into PrP^{Sc} and amplifying PrP^{Sc}, sonicating the resulting product to micronize aggregated PrP^{Sc}, and re-incubating the micronized PrP^{Sc} with excessive PrP^{C}.

The PMCA method is highly effective in the amplification of PrP^{Sc} from a hamster model of scrapie infection. The average amplification factor of PrP^{Sc} after repeating 5 cycles is 58, and repeating 10 cycles allow for detection of even PrP^{Sc} samples diluted 10,000 or more-fold. Thus, a trace amount of PrP^{Sc} can be detected in body fluids such as the blood (Non-Patent Document 3) and the urine (Non-Patent Document 4) obtained from an infected hamster, and also PrP^{Sc} can be detected in blood cells derived from an animal during the incubation period (Non-Patent Documents 4 and 5). Thus, the PMCA method has been shown to be useful as an early diagnostic method of prion diseases and an examination method of prion inactivation.

However, although the proposed PMCA method is highly effective in the amplification of PrP^{Sc} from a hamster model of scrapie infection, it does not provide sufficient amplification when applied to the amplification of the abnormal prion protein (PrP^{Sc}) from bovine spongiform encephalopathy (BSE). Accordingly, the PMCA method is not satisfactorily applicable to an ante-mortem diagnostic method or an early diagnostic method of BSE.
[Patent Document 1] Japanese Patent Application Laid-Open No. 2004-503748
[Non-Patent Document 1] Nature, 383: p685-690 (1996)
[Non-Patent Document 2] Nature, 411: p810-813 (2001)
[Non-Patent Document 3] Nat. Med., 11: p982-985 (2005)
[Non-Patent Document 4] J. Gen. Virol., 88: p2890-2898 (2007)
[Non-Patent Document 5] Science, 313: p92-94 (2006)

### DISCLOSURE OF THE INVENTION

### PROBLEMS TO BE SOLVED BY THE INVENTION

In view of the above circumstances, it is a primary object of the present invention to provide a method for efficiently amplifying abnormal prion protein (PrP^{Sc}) derived from a bovine spongiform encephalopathy (BSE) animal infected with PrP^{Sc}. An ultimate object of the present invention is to eradicate the transmission of a prion disease by detecting a BSE-infected cow early and to develop a method for inactivating prions and to permit early examination of prion inactivation.

### MEANS FOR SOLVING THE PROBLEMS

To solve the above problems, the present inventors enabled abnormal prion protein (PrP^{Sc}) derived from BSE to be amplified efficiently by modifying the conventional PMCA method and accomplished the present invention.

The PMCA method proposed until now, which enables amplification of PrP^{Sc} from a hamster model of scrapie infection, usually amplifies PrP^{Sc} in the presence of a surfactant. The present inventors carried out examinations to improve the method in this respect, and added a polysaccharide sulfate in the amplification of PrP^{Sc} derived from BSE and performed amplification by PMCA. As a result, the present inventors found that the amplification efficiency of PrP^{Sc} was greatly improved due to the effect of the added polysaccharide sulfate, and detection of a trace amount of PrP^{Sc} derived from BSE, which a conventional PMCA method has not successfully detected, was enabled. Thus, the present inventors accomplished the present invention.

Accordingly, the present invention provides a method for efficiently amplifying abnormal prion protein (PrP^{Sc}) derived from bovine spongiform encephalopathy (BSE), wherein the method is based on a PMCA (protein misfolding cyclic amplification) method in which normal prion protein (PrP^{C}) is used as a source and PrP^{Sc} is used as a seed, and PrP^{Sc} derived from BSE is amplified by stir-mixing, incubating, and sonicating both the PrP^{C} and the PrP^{Sc} repeatedly, and wherein
the method includes performing stir-mixing-incubation-sonication in the presence of a polysaccharide sulfate.

Specifically, the present invention provides a method for efficiently amplifying PrP^{Sc} derived from BSE, wherein the PrP^{C} used as a source derives from a brain homogenate containing PrP^{C}, and the PrP^{Sc} derived from BSE used as a seed derives from a body tissue of a BSE-infected animal. Furthermore, the present invention provides a method for efficiently amplifying PrP^{Sc} derived from variant Creutzfeldt-Jakob disease, which is believed to be a disease resulting from infection of humans with BSE.
Thus, the present invention provides not only efficient amplification of abnormal prion protein derived from a BSE-infected animal but also a method for efficiently amplifying PrP^{Sc} derived from BSE, the PrP^{Sc} deriving from a living tissue including that of a human suffering from prion disease caused by BSE infection.

More specifically, the present invention includes the following embodiments:
(1) a method for efficiently amplifying PrP^{Sc} derived from BSE as described above, wherein the polysaccharide sulfate is a polysaccharide sulfate which includes a sulfate group carrying a negative charge in a solution;
(2) a method for efficiently amplifying PrP^{Sc} derived from BSE as described above, wherein the polysaccharide sulfate which includes a sulfate group carrying a negative charge in a solution is dextran sulfate or pentosan polysulfate;
(3) a method for efficiently amplifying PrP^{Sc} derived from BSE as described above, wherein the polysaccharide sulfate is a polysaccharide sulfate with a molecular weight of 5 to 6 KD;
(4) a method for efficiently amplifying PrP^{Sc} derived from BSE as described above, wherein the polysaccharide sulfate is a polysaccharide sulfate with a molecular weight of 1.5 to 1.9 KD;
(5) a method for efficiently amplifying PrP^{Sc} derived from BSE as described above, wherein the concentration of added polysaccharide sulfate is 0.005 to 1%;
(6) a method for efficiently amplifying PrP^{Sc} derived from BSE as described above, wherein the polysaccharide sulfate is dextran sulfate or pentosan polysulfate; and
(7) a method for efficiently amplifying PrP^{Sc} derived from BSE as described above, wherein the dextran sulfate is dextran sulfate sodium or dextran sulfate potassium.

### EFFECTS OF THE INVENTION

The method of the present invention enables a trace amount of PrP^{Sc} derived from BSE to be detected. The sensitivity in detecting PrP^{Sc} derived from BSE by using the method of the present invention is remarkably efficient compared to preexisting detection methods such as the ELISA method. Thus, the method of the present invention is advantageous in that the sensitivity obtained after one round of amplification is higher than that in a bioassay.
Moreover, the method of the present invention can avoid spending an enormous amount of time and money, which a conventional bioassay requires for detection of PrP^{Sc}, and thus it also has excellent practicality and rapidity.

Accordingly, the method of the present invention permits an ante-mortem diagnosis and an early diagnosis of BSE, and moreover, it permits rapid examination of inactivation of abnormal prion protein derived from BSE, thereby contributing to establishing a method for inactivating abnormal prion protein. This method also has the advantage that it is applicable to a safety evaluation method of raw materials for feed and fertilizer such as meat and bone meal, environmental monitoring of, for example, PrP^{Sc} in soil and the like.
In particular, the occurrence of BSE has been confirmed not only in Japan but also all over the world, mainly in the EU region, and the risk of BSE occurring in the future should be taken into consideration in countries with no existing BSE. The method of the present invention is useful as a communicable disease control measure for BSE for organism (animal) import and also as a preventive measure against BSE for imported beef.

### BRIEF DESCRIPTION OF THE DRAWINGS

Fig. 1 shows the results of amplification in the presence of different additives in accordance with Test Example 1 of the present invention.
Fig. 2 shows the results of examination of the addition of Fucoidan in accordance with Test Example 2 of the present invention.
Fig. 3 shows the results of examination of the addition of chondroitin sulfate sodium (CSS) and λ-carragheenan (Cag) in accordance with Test Example 2 of the present invention.
Fig. 4 shows the results of examination of the addition of heparan sulfate sodium (HSS), heparan sulfate proteoglycan (HSPG), and polyvinyl sulfate sodium (PVSP) in accordance with Test Example 2 of the present invention.
Fig. 5 shows the results of examination of the addition of pentosan polysulfate (PPS) in accordance with Test Example 2 of the present invention.

Fig. 6 shows the results of examination of the addition of dextran sulfate sodium (DSS) in accordance with Test Example 3 of the present invention.
Fig. 7 shows the results of examination of the amounts of dextran sulfate potassium (DSP) to be added in accordance with Test Example 4 of the present invention.
Fig. 8 shows the results of examination of the amounts of dextran sulfate potassium (DSP) at low concentrations to be added in accordance with Test Example 4 of the present invention.
Fig. 9 shows the results of examination of different cycles of incubation-sonication process in accordance with Test Example 5 of the present invention.
Fig. 10 shows the PrP^{Sc} distribution in peripheral nerve tissues analyzed by using an experimentally BSE-infected cow in accordance with Test Example 6 of the present invention.

### BEST MODE FOR CARRYING OUT THE INVENTION

As described above, the basic embodiment of the present invention is a method for efficiently amplifying abnormal prion protein (PrP^{Sc}) derived from BSE, wherein the method is based on a PMCA method in which normal prion protein (PrP^{C}) is used as a source and PrP^{Sc} is used as a seed, and PrP^{Sc} from BSE is amplified by stir-mixing, incubating, and sonicating both PrP^{C} and PrP^{Sc} repeatedly, and wherein
the method includes performing stir-mixing-incubation-sonication in the presence of a polysaccharide sulfate.

Body tissue of a normal cow can be used to provide normal prion protein (PrP^{C}) which is used as a source in this method. Preferably, a brain homogenate of a normal cow is used.
The brain homogenate is prepared by grinding (homogenating) the brain of a normal cow, for example in a mortar. More specifically, 10% (w/v) suspension that is prepared by homogenizing the brain of a normal cow in PBS with 1% Triton X-100 (t-octylphenoxy polyethoxyethanol) and 4 mM EDTA (containing a protease inhibitor) is preferably used.

Also, a brain homogenate of a heterologous animal carrying an introduced cow PrP gene, for example, a cow PrP-transgenic mouse, or alternatively, a diluted brain homogenate that is prepared by mixing this brain homogenate with a brain homogenate of a cow PrP-knockout mouse may be used.

On the other hand, body tissue of a BSE-infected cow is preferably used to provide PrP^{Sc} derived from BSE which is used as a seed. Any body tissue, for example, brain tissue, blood tissue, urine derived from a BSE-infected cow can be used as such a body tissue.
Thus, the amplification method provided by the present invention enables even a trace amount of PrP^{Sc} to be amplified efficiently. Accordingly, the novel PMCA method permits detection of samples containing a trace amount of abnormal prion protein derived from BSE and increases the sensitivity in detecting a BSE-infected cow. Thus, PrP^{Sc} derived from BSE which is used as a seed is not particularly limited and any body tissue derived from a BSE-infected cow can be used.

Additionally, PrP^{Sc} derived from BSE which is used as a seed is not limited to those above described and may include PrP^{Sc} derived from variant Creutzfeldt-Jakob disease, which is believed to be a disease resulting from infection of humans with BSE. In other words, not only body tissue derived from a BSE-infected cow but also a living tissue including that of a human suffering from prion disease caused by BSE infection may be used.

Incidentally, when amplification is performed according to the conventional PMCA method for amplifying PrP^{Sc} from a hamster model of scrapie infection, amplification is usually achieved by adding a surfactant to abnormal prion protein and solubilizing PrP^{Sc}, and then mixing the solubilized PrP^{Sc} with PrP^{C} allowing conformational conversion of PrP^{C} used as a source into PrP^{Sc}.
The PMCA method of the present invention is also performed in the presence of a surfactant. Such surfactant is a non-ionic surfactant, and preferably t-octylphenoxy polyethoxyethanol (Triton X-100), polyoxyethylene (9) octylphenyl ether (NP-40) or the like is used without limitation.
However, for PrP^{Sc} derived from BSE, an efficient amplification reaction of PrP^{Sc} was not achieved by addition of a surfactant alone in this step.

In the method provided by the present invention, an efficient amplification reaction of PrP^{Sc} was achieved by adding a polysaccharide sulfate in addition to a surfactant.
Although the rationale is not certain, one possible explanation is that this happens since a polysaccharide sulfate has a strong action to deprive hydration water covering the surface of the proteins, and thus the molecular bond between the prion proteins (normal prion protein and abnormal prion protein) is strengthened. Another possible explanation is that the polysaccharide sulfate binds to a prion protein and induces a protein structure suitable for amplification.
Accordingly, the modified PMCA method according to the present invention includes mixing PrP^{Sc} derived from BSE with PrP^{C} and incubating with stirring the mixture to allow conformational conversion of PrP^{C} into PrP^{Sc}, and a polysaccharide sulfate is believed to act effectively in this conformational conversion process.

Such polysaccharide sulfates to be added include polysaccharide sulfates which include a sulfate group carrying a negative charge in a solution.
The inventor's studies showed that little effect was observed when a positively-charged DEAE (dimethylaminoethyl)-dextran compound or uncharged dextran itself was used (see Test Examples described later).
Such polysaccharide sulfates which include a sulfate group carrying a negative charge in a solution may include dextran sulfates or pentosan polysulfates, particularly dextran sulfates such as dextran sulfate sodium and dextran sulfate potassium.

In the method provided by the present invention, the amplification effect of PrP^{Sc} was also found to be related to the molecular weight of the polysaccharide sulfate to be added.
For example, when dextran sulfate was used as a polysaccharide sulfate, the amplification efficiency was higher with low-molecular-weight dextran sulfates such as dextran sulfate sodium with a molecular weight of 5 to 6 KD or dextran sulfate potassium with a molecular weight of 1.5 to 1.9 KD, compared to high-molecular-weight dextran sulfate sodium with a molecular weight of 900 to 2,000 KD (see Test Examples described below).

The concentration of the polysaccharide sulfate added with a surfactant also affects the amplification efficiency. For example, when dextran sulfate was used, it was found that an appropriate concentration of added dextran sulfate was about 0.005 to 1%, and concentrations not more than or not less than the appropriate concentration cause decrease in the amplification efficiency (see Test Examples described below).

Incidentally, polysaccharide sulfates, which are not dextran sulfate but similar thereto, the dextran sulfate being based on polymerized glucose molecules, for example, Fucoidan, λ-carrageenan, or sulfated glycosaminoglycan in a living body, for example, chondroitin sulfate sodium, heparan sulfate sodium, and heparan sulfate proteoglycan did not increase the amplification effect as much as dextran sulfate.
On the other hand, polyvinyl sulfate sodium, which is an anionic polymer, was not effective, either (see studies described below).
In this respect, the addition of polysaccharide sulfate used in the method of the present invention may be considered to be highly effective for the amplification of PrP^{Sc} derived from BSE.

The general operational procedures of the method of the present invention will be described below.
That is, the method of the present invention uses the above-mentioned PrP^{C} as a source and PrP^{Sc} derived from BSE as a seed, and stir-mixes and incubates both PrP^{C} and PrP^{Sc}. The incubating (incubation) condition is not necessarily limited and an optimal incubation condition may be selected as appropriate. Specifically, for example, the method may include incubation for about one hour at 37°C with stirring.

This stir-mixing and incubation allows the conformational conversion of Pr^{C} added as a source into PrP^{Sc} in part. The aggregates of conformationally converted PrP^{Sc} are dispersed by sonication and the cycle of stir-mixing and incubation with excessive Pr^{C} is repeated again.
The sonication is not particularly limited and the sonication used in the usual PMCA method may be used without any change. For example, sonication may be performed on Digital Sonifier 450D (Branson) or 070-GOT (ELECON Corporation).
Although sonication conditions may vary with the systems used and are not necessarily limited, an exemplary condition is, for example, about 5 cycles of a 0.2 second oscillation and a 0.1 second pause or a 3 second oscillation and a 1 second pause with output setting at 100%.

While the cycles of stir-mixing-incubation-sonication are repeated, PrP^{C} added as a source is conformationally converted to PrP^{Sc} sequentially, so that PrP^{Sc} is amplified.
To achieve one round of amplification by the method of the present invention, in general, the cycles described above are preferably performed 20 to 40 times per one round of amplification process.
Then, the number of cycles depends on the concentrations of PrP^{C} used as a source and of PrP^{Sc} used as a seed and is not limited.

After the amplification (repeating the cycles of incubation with stirring and sonication) of PrP^{Sc} is completed, the reactant obtained is subjected to a degradation process using proteases for degradation.
Since abnormal prion protein is generally resistant to proteases, it is necessary to degrade normal prion protein to recover this protein specifically. Thus, this degradation process is a process in which proteins other than the prion protein are degraded and additionally the normal prion protein is also degraded.
Proteases may include proteinase K. It is desirable to use proteinase K for degradation.

Incidentally, amplified PrP^{Sc} can be detected by Western blotting. Specifically, a sample is electrophoresed and separated on 15% SDS-PAGE and transferred to a membrane. After blocking, the membrane is reacted with HRP-labelled anti-T2 antibodies. Subsequently, after washing the membrane, amplified PrP^{Sc} can be detected and identified by detecting a luminous reaction using Immobilon Western.

The present inventors' studies demonstrated that one round of amplification (40 cycles of incubation with stirring and sonication) provides a higher sensitivity than that of a bioassay.
Thus, the method of the present invention has extremely good practicality and rapidity, has a great possibility of being used as a diagnosis, a safety evaluation method, and a preventive measure regarding BSE, and has extensive applicability.

### [EXAMPLES]

Herein below, the present invention will be described in more detail with reference to Test Examples instead of Implementation Examples, but these Test Examples are not meant to limit the present invention in any way.

### Test Example 1: Amplification of PrP^{Sc} Derived from BSE (Examination

### of Additives 1)

### 1. Method

Abnormal prion protein (PrP^{Sc}) derived from BSE was amplified using a PMCA (protein misfolding cyclic amplification) method.
A normal prion protein (PrP^{C}) source was prepared by 8-fold diluting a brain homogenate of a transgenic mouse (a TgBo mouse) carrying an introduced cow prion gene with a brain homogenate of a prion gene-knockout mouse and adding different additives at a final concentration of 0.5%.
On the other hand, a 10⁻² to 10⁻¹⁰-fold dilution prepared from a BSE-infected brain homogenate from the United Kingdom (infectivity titer: 10^{6.7} LD₅₀/g; Jpn. J. Infect. Dis., 60: p317-320 (2007)) was used as a PrP^{Sc} seed.
After PMCA amplification, the samples were digested by proteinase K and the signal from protease-resistant PrP (PrP^{RES}) was detected by Western blotting.

Dextran sulfate sodium with a molecular weight of 900 to 2,000 KD (DSS 900-2,000KD), dextran sulfate sodium with a molecular weight of 5 to 6 KD (DSSII 5-6KD), dextran sulfate potassium with a molecular weight of 1.5 to 1.9 KD (DSP 1.6-1.9KD), DEAE-dextran with a molecular weight of 50 KD (DEAE-Dextran 50KD), and dextran (Dextran) that has various molecular weights (15 to 230 KD) were used as additives.
Additionally, a comparative amplification with no additives was performed as a control.

### 2. Result

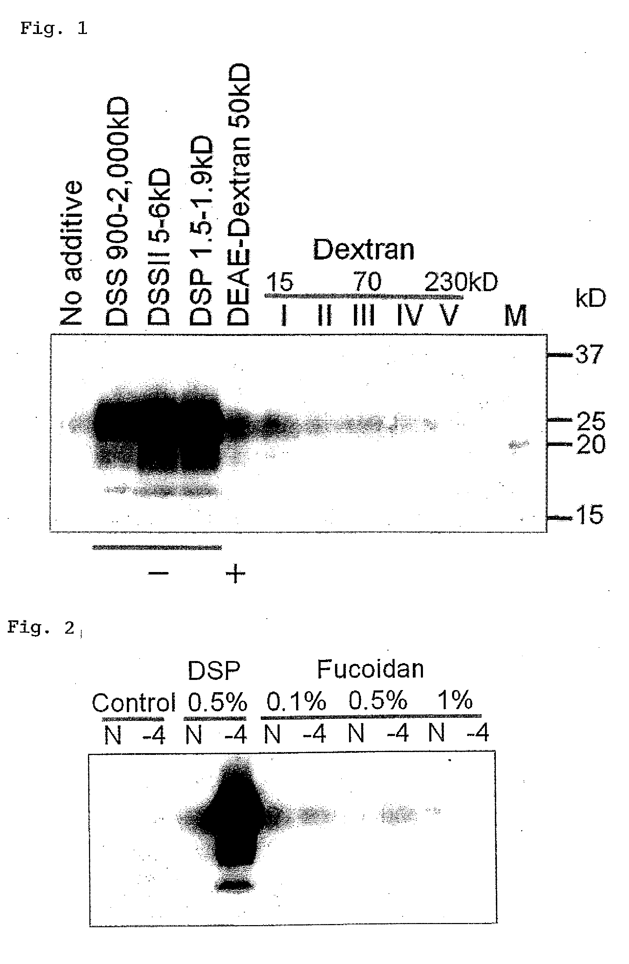

The results are shown in Fig. 1. As is understood from the results shown in Fig. 1, it was found that good amplification of PrP^{Sc} was observed when dextran sulfate sodium or dextran sulfate potassium, which is a polysaccharide sulfate that includes a sulfate group carrying a negative charge in a solution, was added as an additive.
In contrast, little amplification of PrP^{Sc} was observed when the control with no additives, positively charged DEAE-dextran, or uncharged dextrans of various molecular weights was used as an additive. These results strongly show the efficacy of the present invention.
Furthermore, it was found that among dextran sulfates that showed an amplification effect, low-molecular-weight dextran sulfates such as dextran sulfate sodium with a molecular weight of 5 to 6 KD or dextran sulfate potassium with a molecular weight of 1.5 to 1.9 KD (DSP 1.6-1.9KD) led to a higher-amplification efficiency, compared to high-molecular-weight dextran sulfate sodium with a molecular weight of 900 to 2,000 KD.

### Test Example 2: Amplification of PrP^{Sc} derived from BSE (Examination of Additives 2)

The results from Test Example 1 demonstrated that the addition of dextran sulfate was highly effective for the amplification of PrP^{Sc}. The present inventors then examined if the addition of other compounds having a sulfate group was effective for the amplification of PrP^{Sc}.

### 1. Method

PrP^{Sc} derived from BSE was amplified using the PMCA method as with Test Example 1.
In addition to dextran sulfate potassium (DSP) and pentosan polysulfate (PPS) as a polysaccharide sulfate, Fucoidan, chondroitin sulfate sodium (CSS), λ-carragheenan (Cag), heparan sulfate sodium (HSS), and heparan sulfate proteoglycan (HSPG), which are polysaccharide sulfates, and polyvinyl sulfate sodium (PVSP), which is an anionic polymer, were used as additives.

### [A] Examination of the Addition of Fucoidan:

When Fucoidan was added, a 10⁻⁴-fold dilution of a BSE-infected brain homogenate was used as a PrP^{Sc} seed. Each sample was prepared by adding dextran sulfate potassium (DSP) at a final concentration of 0.5% or Fucoidan at a final concentration of 0.1%, 0.5%, or 1.0% to the dilution. A sample with no added seed and a sample with no additives served as controls for comparison.

### [B] Examination of the Addition of Chondroitin Sulfate Sodium (CSS) and λ-Carragheenan (Cag):

A 10⁻⁴-fold dilution of a BSE-infected brain homogenate was used as a PrP^{Sc} seed. Each sample was prepared by adding dextran sulfate potassium (DSP) at a final concentration of 0.05% or 0.5% or chondroitin sulfate sodium (CSS) or λ-carragheenan (Cag) at a final concentration of 0.05% or 0.5% to the dilution. A sample with no additives served as a control for comparison.

### [C] Examination of the Addition of Heparan Sulfate Sodium (HSS), Heparan Sulfate Proteoglycan (HSPG), and Polyvinyl Sulfate Sodium (PVSP):

A 10⁻⁴-fold dilution of a BSE-infected brain homogenate was used as a PrP^{Sc} seed. Each sample was prepared by adding dextran sulfate potassium (DSP), heparan sulfate sodium (HSS), heparan sulfate proteoglycan (HSPG), or polyvinyl sulfate sodium (PVSP), each at a final concentration of 0.5%, to the dilution.
Additionally, a sample with no added seed and a sample with no additives served as controls for comparison.

### [D] Examination of the Addition of Pentosan Polysulfate (PPS):

A 2 x 10⁻⁴-fold dilution of a BSE-infected brain homogenate was used as a PrP^{Sc} seed. Each sample was prepared by adding 50 µg/mL or 500 µg/mL of dextran sulfate potassium (DSP), or 0.5 µg/mL, 5 µg/mL, 50 µg/mL, or 500 µg/mL of pentosan polysulfate (PPS) to the dilution.
Additionally, a sample with no added seed and a sample with no additives served as controls for comparison.

### 2. Results

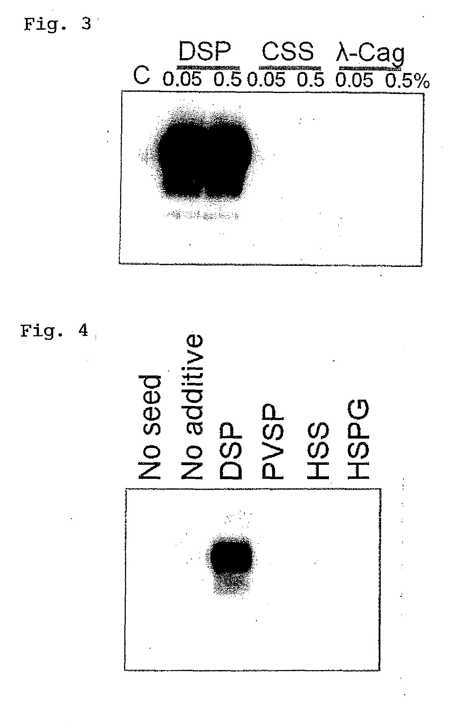

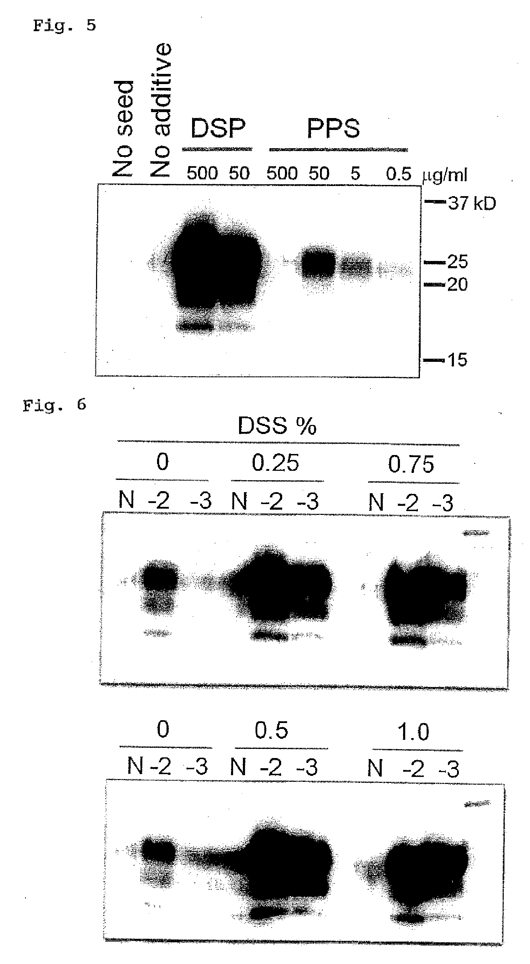

Fig. 2 shows the results of examination of the addition of Fucoidan, Fig. 3 shows the results of examination of the addition of chondroitin sulfate sodium (CSS) and λ-carragheenan (Cag), Fig. 4 shows the results of examination of the addition of heparan sulfate sodium (HSS), heparan sulfate proteoglycan (HSPG), and polyvinyl sulfate sodium (PVSP), and Fig. 5 shows the results of examination of the addition of pentosan polysulfate (PPS).
As is also understood from the results shown in Figs. 2 to 5, significant amplification of PrP^{Sc} was observed when dextran sulfate potassium or pentosan polysulfate, which is a polysaccharide sulfate, was added.
In contrast, although Fucoidan, chondroitin sulfate sodium (CSS), λ-carragheenan (Cag), heparan sulfate sodium (HSS), and heparan sulfate proteoglycan (HSPG) are compounds that have a sulfate group, they had a lower amplification effect than dextran sulfate and pentosan polysulfate.

### Test Example 3: Amplification of PrP^{Sc} Derived from BSE (Examination of Concentrations of an Additive 1)

The results from Test Examples 1 and 2 demonstrated that the addition of dextran sulfate as a polysaccharide sulfate was highly effective for the amplification of PrP^{Sc} derived from BSE. Thus, the present inventors then examined how a final concentration of the additive affected the amplification of PrP^{Sc}.

### 1. Method

PrP^{Sc} derived from BSE was amplified using the PMCA method as with studies described above.
A normal prion protein (PrP^{C}) source was prepared by 8-fold diluting a brain homogenate of a transgenic mouse (a TgBo mouse) carrying an introduced cow prion gene with a brain homogenate of a prion gene-knockout mouse, and adding dextran sulfate sodium (DSS) to a final concentration of 0%, 0.25%, 0.5%, 0.75%, or 1.0%.
A 10⁻²-fold dilution and a 10⁻³-fold dilution prepared from a BSE-infected brain homogenate from the United Kingdom (infectivity titer: 10^{6.7} LD₅₀/g) were used as a PrP^{Sc} seed as with Test Example 1.
Additionally, a sample with no added PrP^{Sc} seed served as a control for comparison.
After PMCA amplification, the samples were digested by proteinase K and the signal from protease-resistant PrP (PrP^{RES}) was detected by Western blotting.

### 2. Results

The results are shown in Fig. 6. As is also understood from the results in Fig. 6, although the amplification level of PrP^{Sc} in the absence of dextran sulfate sodium was low, the amplification efficiency of PrP^{Sc} dramatically increased when dextran sulfate sodium was added.
Dextran sulfate sodium that was added at a concentration ranging about 0.25% to 1% was found to produce a sufficient amplification effect.

### Test Example 4: Amplification of PrP^{Sc} Derived from BSE (Examination of Concentrations of an Addictive 2)

The results from Test Example 3 demonstrated that dextran sulfate sodium (DSS) as an additive, which is a polysaccharide sulfate, increased the amplification efficiency of PrP^{Sc} when it was added at a concentration ranging from about 0.25% to 1%.
Subsequently, dextran sulfate potassium (DSP), which is a polysaccharide sulfate, was used as an additive and an optimal concentration thereof was determined.

### 1. Method

This Test Example was performed as with Test Example 3.
Firstly, dextran sulfate potassium (DSP) was added at concentrations of 0.5%, 1.0%, and 2.0%, and the amplification effect was examined. Considering that dextran sulfate sodium added at 0.5% or less was found to produce a sufficient amplification effect, dextran sulfate potassium was then added at concentrations of 0.05%, 0.005%, 0.0005%, and 0.00005%, and the amplification effect was examined.
Additionally, a sample with no added PrP^{Sc} seed and a sample with no additives served as controls for comparison.

### 2. Results

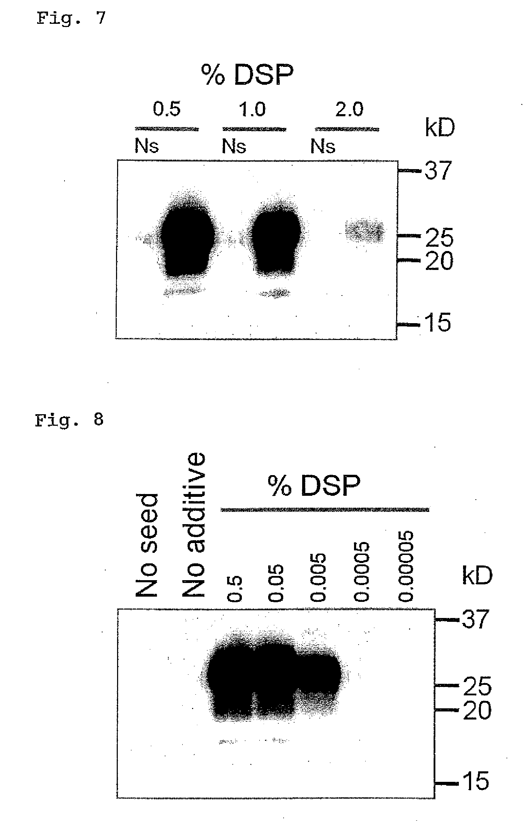

The results are shown in Figs. 7 and 8.
Fig. 7 shows the results of examination of the amount of dextran sulfate potassium (DSP), which was added at concentrations of 0.5%, 1.0%, and 2.0%. Dextran sulfate potassium added at a concentration of 2% or more was found to decrease the amplification of PrP^{Sc} conversely.
Fig. 8 shows the results of examination of the amount of dextran sulfate potassium (DSP), which was added at a concentration of 0.5% or less. Dextran sulfate potassium added at as low a concentration as 0.005% was found to produce a sufficient amplification of PrP^{Sc}, but dextran sulfate potassium of 0.005% or less did not produce an amplification effect.
It is understood from the results above that in the present invention, the optimal concentration of a polysaccharide sulfate to be added is about 0.005 to 1% and a polysaccharide sulfate whose concentration is not more than or not less than the optimal concentration decreases the amplification efficiency.

### Test Example 5: Amplification of PrP ^{Sc} Derived from BSE (Examination of Cycles of Incubation-Sonication)

In the PMCA, method of the present invention, cycles of incubation-sonication are repeated and one round of amplification of PrP^{Sc} is completed by repeating 40 cycles.
Thus, the difference of amplification effect for PrP^{Sc} produced by the different number of cycles was examined.

### 1. Method

PrP^{Sc} derived from BSE was amplified using the PMCA method as with Test Example 1 described above.
A normal prion protein (PrP^{C}) source was prepared by 8-fold diluting a brain homogenate of a transgenic mouse (a TgBo mouse) carrying an introduced cow prion gene with a brain homogenate of a prion gene-knockout mouse and adding dextran sulfate potassium at a final concentration of 0.5%.
On the other hand, a 10⁻² to 10⁻¹⁰ dilution prepared from a BSE-infected brain homogenate from the United Kingdom (infectivity titer: 10^{6.7} LD₅₀/g; Jpn. J. Infect. Dis., 60: p317-320 (2007)) was used as a PrP^{Sc} seed.
After PMCA amplification, the samples were digested by proteinase K and the signal from protease-resistant PrP (PrP^{RES}) was detected by Western blotting.

### 2. Results

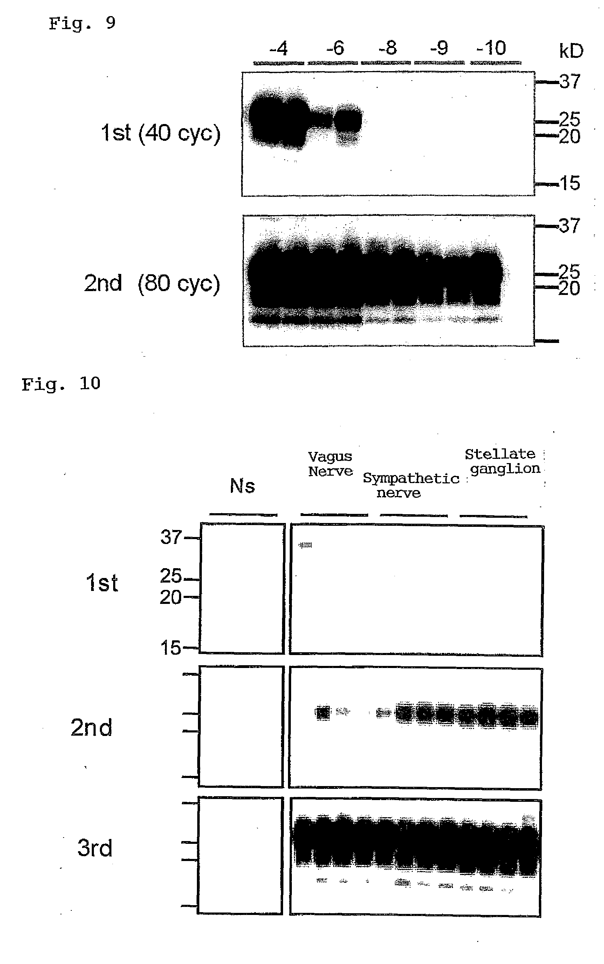

The results are shown in Fig. 9.
As is also understood from the results shown in Fig. 9, the signal from PrP^{RES} was observed in duplicate samples that were 10⁻⁶-fold dilutions after one round of amplification (40 cycles).
Since the detection limit for infectivity of the infected brain homogenate used for PMCA amplification is known to be 10⁻⁴-fold, it can be concluded that a sensitivity that is nearly 100 times the sensitivity of a bioassay was obtained by one round, of amplification.
Furthermore, when the PMCA product was 1/5 diluted with a PrP^{Sc} source and a second round of amplification was performed (80 cycles), the PrP^{RES} signal was detected in both of the 10⁻⁹-fold diluted duplicate samples and in one of the 10⁻¹⁰-fold diluted duplicate samples.
Although the detection limit with which a conventional PMCA method can detect PrP^{Sc} in a hamster model of scrapie infection is about 10⁻¹²-fold, it requires 6 to 7 repeats of amplification to achieve this. The infectivity titer of the BES-infected brain homogenate used in this Test Example was about one-hundredth the infectivity titer of a brain homogenate derived from an experimental hamster model of infection. Considering this respect, as little as 2 rounds of amplification resulted in a detection sensitivity for PrP^{Sc} comparable to the detection limit in the hamster model of scrapie infection.

### Test Example 6: Detection of PrP^{Sc} in a Peripheral Tissue of an Experimentally BSE-Infected Cow

The present invention enabled detection of a trace amount of PrP^{Sc} derived from BSE and more detailed analysis of bio-kinetics of PrP^{Sc} in an infected cow compared to a conventional method. The PrP^{Sc} distribution in a peripheral nerve tissue was then analyzed by using an experimentally BSE-infected cow.

### 1. Method

PrP^{Sc} derived from an experimentally BSE-infected cow was amplified using the PMCA method as with Test Example 1 described above. A normal prion protein (PrP^{C}) source was prepared by 5-fold diluting a 10% brain homogenate of a transgenic mouse (a TgBo mouse) carrying an introduced cow prion gene with a 10% brain homogenate of a prion gene-knockout mouse and adding dextran sulfate potassium at a final concentration of 0.5%. On the other hand, a 20% homogenate was prepared from a peripheral nerve tissue, 1/20 diluted with the PrP^{C} source, and used as a PrP^{Sc} seed. After PMCA amplification, the samples were digested by proteinase K and the signal from protease-resistant PrP (PrP^{RES}) was detected by Western blotting. Amplification was performed with quadruplet samples of each tissue.

### 2. Results

The results are shown in Fig. 10.
The BSE-infected cow used in this experiment was dissected 36 months after oral inoculation and each tissue was removed. BSE had not developed at the time of dissection and PrP^{Sc} was not detected in peripheral tissues by usual Western blotting.
As is understood from the results shown in the figure, the signal was detected in a vagus nerve, a sympathetic nerve, and a stellate ganglion after 2 rounds of amplification. Furthermore, the PrP^{Sc} signal was enhanced in all the samples after the third round of amplification and the presence of PrP^{Sc} became clear.
The results show the amplification results for each peripheral nerve tissue (in quadruplicate samples). Ns in the figure refers to a control with no added seed.

It became apparent from the results from this experiment that the method according to the present invention for amplifying PrP^{Sc} derived from BSE was also effective in the samples from peripheral tissue and enabled PrP^{Sc} to be detected in cows that had not developed BSE, and thus was applicable to an early identification of a BSE-infected cow.

### INDUSTRIAL APPLICABILITY

As described above, the method provided by the present invention enables an effective amplification of PrP^{Sc} derived from BSE and detection of a trace amount of PrP^{Sc}.
The sensitivity in detecting PrP^{Sc} derived from BSE by using the method of the present invention is remarkably efficient compared to existing detection methods such as the ELISA method. The method of the present invention is advantageous in that the sensitivity obtained after one round of amplification is higher than that in a bioassay, and thus it also has excellent practicality and rapidity.

Accordingly, the method of the present invention permits an ante-mortem diagnosis and an early diagnosis of BSE, and moreover, it permits rapid examination of inactivation of abnormal prion protein derived from BSE, thereby contributing to establishing a method for inactivating abnormal prion protein.
This method is also applicable to a safety evaluation method of raw materials for feed and fertilizer such as meat and bone meal, environmental monitoring of, for example, PrP^{Sc} in soil and the like, and furthermore, a communicable disease control measure for BSE for organism (animal) import, and furthermore, a preventive measure against BSE for imported beef. Thus, this method is highly useful.

## Claims

1. A method for efficiently amplifying abnormal prion protein (PrP^{Sc}) derived from bovine spongiform encephalopathy (BSE), wherein the method is based on a PMCA (protein misfolding cyclic amplification) method in which normal prion protein (PrP^{C}) is used as a source and PrP^{Sc} is used as a seed, and PrP^{Sc} derived from BSE is amplified by stir-mixing, incubating, and sonicating both the PrP^{C} and the PrP^{Sc} repeatedly, and
the method comprising performing stir-mixing-incubation-sonication in the presence of a polysaccharide sulfate.

2. The method for efficiently amplifying PrP^{Sc} derived from BSE according to claim 1, wherein the PrP^{C} used as a source derives from a brain homogenate containing PrP^{C}, and the PrP^{Sc} derived from BSE used as a seed derives from a body tissue containing PrP^{Sc} derived from a BSE-infected animal or PrP^{Sc} derived from variant Creutzfeldt-Jakob disease resulting from infection with BSE.

3. The method for efficiently amplifying PrP^{Sc} derived from BSE according to claim 1, wherein the polysaccharide sulfate is a polysaccharide sulfate which includes a sulfate group carrying a negative charge in a solution.

4. The method for efficiently amplifying PrP^{Sc} derived from BSE according to claim 1, wherein the polysaccharide sulfate which includes a sulfate group carrying a negative charge in a solution is dextran sulfate or pentosan polysulfate.

5. The method for efficiently amplifying PrP^{Sc} derived from BSE according to claim 1, wherein the polysaccharide sulfate is a polysaccharide sulfate with a molecular weight of 5 to 6 KD.

6. The method for efficiently amplifying PrP^{Sc} derived from BSE according to claim 1, wherein the polysaccharide sulfate is a polysaccharide sulfate with a molecular weight of 1.5 to 1.9 KD.

7. The method for efficiently amplifying PrP^{Sc} derived from BSE according to claim 1, wherein a concentration of the polysaccharide sulfate added is 0.005 to 1%.

8. The method for efficiently amplifying PrP^{Sc} derived from BSE according to claim 1, wherein the polysaccharide sulfate is dextran sulfate or pentosan polysulfate.

9. The method for efficiently amplifying PrP^{Sc} derived from BSE according to claim 8, wherein the dextran sulfate is dextran sulfate sodium or dextran sulfate potassium
